# EUROPÄISCHE PATENTSCHRIFT

(11) **EP 1 348 458 B1**
(45) Veröffentlichungstag und Bekanntmachung des Hinweises auf die Patenterteilung: **23.11.2005**
(21) Anmeldenummer: 03001930.1
(22) Anmeldetag: 30.01.2003
(51) Int. Cl.: A61M 1/34, A61M 1/36

(54) **Verfahren und Vorrichtung zur Überwachung der Zufuhr von Substitutionsflüssigkeit während einer extrakorporalen Blutbehandlung**
Method and device for supervising the supply of substitution fluid during an extracorporeal blood treatment
Méthode et dispositif pour surveiller l'adduction de liquide de substitution pendant un traitement extracorporel du sang

(30) Priorität: 25.03.2002 DE 10213179
(43) Veröffentlichungstag der Anmeldung: 01.10.2003
(73) Patentinhaber: Fresenius Medical Care Deutschland GmbH, 61352 Bad Homburg v.d.H. (DE)
(72) Erfinder: Zhang, Wei, 97421 Schweinfurt (DE)
(74) Vertreter: Luderschmidt, Schüler & Partner GbR

(56) Entgegenhaltungen:
- EP-A- 0 189 561
- EP-A- 1 095 666
- EP-A- 1 175 917
- US-A- 5 366 630

## Beschreibung

Die Erfindung betrifft ein Verfahren zur Detektion der Zufuhr von Substitutionsflüssigkeit aus dem Flüssigkeitssystem einer extrakorporalen Blutbehandlungsvorrichtung in den extrakorporalen Blutkreislauf stromauf oder stromab des Dialysators oder Filters der Blutbehandlungsvorrichtung. Darüber hinaus bezieht sich die Erfindung auf eine Vorrichtung zur extrakorporalen Blutbehandlung mit einer Einrichtung zur Detektion der Zufuhr von Substitutionsflüssigkeit stromauf oder stromab des Dialysators oder Filters.

Zur Entfernung von hampflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt das Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinenmolekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators oder stromab des Dialysators dem extrakorporalen Blutkreislauf zugeführt wird. Die Zufuhr der Substitutionsflüssigkeit stromauf des Dialysators wird auch als Prädilution und die Zufuhr stromab des Dialysators als Postdilution bezeichnet

Es sind Vorrichtungen zur Hämo(dia)filtration bekannt, bei denen die Dialysierflüssigkeit online aus Frischwasser und Konzentraten und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Prädilution führt die Substitutionsflüssigkeitsleitung zu einer Anschlussstelle an der arteriellen Blutleitung stromauf des Dialysators oder Filters, während bei der Postdilution die Substitutionsflüssigkeitsleitung zu einer Anschlussstelle an der venösen Blutleitung stromab des Dialysators führt. Die Susbstitutionsflüssigkeitsleitung weist im Allgemeinen einen Konnektor auf, mit dem sie entweder an die venöse oder arterielle Blutleitung angeschlossen werden kann. Zum Unterbrechen der Flüssigkeitszufuhr ist an der Substitutionsflüssigkeitsleitung eine Klemme oder dgl. vorgesehen. Ein derartiges Hämo(dia)filtrationsgerät ist beispielsweise aus der EP-A-0 189 561 bekannt.

Die Überwachung der Blutbehandlung setzt die Kenntnis voraus, ob die Substitutionsflüssigkeit stromauf oder stromab des Dialysators oder Filters dem extrakorporalen Blutkreislauf zugeführt wird. Beispielsweise spielt die Prä- und Postdilution eine Rolle für die auf einer Leitfähigkeitsmessung beruhenden Online-Clearance-Messung (OCM), da die Leitfähigkeit der Dialysierflüssigkeit stromab des Dialysators davon abhängig ist, ob eine Prä- oder Postdilution erfolgt.

Der Erfindung liegt daher die Aufgabe zugrunde, ein Verfahren und eine Vorrichtung zur zuverlässigen Erkennung einer Prä- und Postdilution anzugeben.

Die Lösung dieser Aufgabe erfolgt erfindungsgemäß mit den im Patentanspruch 1 bzw. 11 angegebenen Merkmalen.

Das erfindungsgemäße Verfahren beruht auf einer Laufzeitmessung der von der Substituatpumpe erzeugten Druckwellen, die Substitutionsflüssigkeit in den extrakorporalen Blutkreislauf fördert. Es hat sich gezeigt, dass die Laufzeit der Druckwellen der Substituatpumpe von der Substituatpumpe bis in den extrakorporalen Blutkreislauf davon abhängig ist, wo die Substitutionsflüssigkeit dem Blutkreislauf zugefiihrt wird, d. h. stromauf oder stromab des Dialysators oder Filters. Dies ist auf die unterschiedlichen Ausbreitungswege der Druckwellen über das Leitungssystem der Blutbehandlungsvorrichtung zurückzuführen.

Zur Bestimmung der Laufzeit der Druckwellen der Substituatpumpe wird vorzugsweise der Druck im extrakorporalen Kreislauf stromab des Dialysators oder Filters gemessen. Damit ergeben sich zwei Ausbreitungswege unterschiedlicher Länge, nämlich einen Ausbreitungsweg, der einen Teil des extrakorporalen Blutkreislaufs stromab des Dialysators oder Filters einschließt, und einen Ausbreitungsweg, der einen Teil des Blutkreislaufs stromauf des Dialysators oder Filters, den Dialysator oder Filter und einen Teil des Blutkreislaufs stromab des Dialysators einschließt.

Die Laufzeit der Druckwellen der Substituatpumpe wird vorzugsweise mit einem vorgegebenen Grenzwert verglichen, der für die Laufzeit über die unterschiedlichen Ausbreitungswege charakteristisch ist. Bei Überschreiten des Grenzwertes wird auf eine Prädilution und bei Unterschreiten des Grenzwertes auf eine Postdilution geschlossen.

In Versuchen hat sich gezeigt, dass die Laufzeit der Druckwellen der Substituatpumpe bei einer Zufuhr von Dialysierflüssigkeit stromauf des Dialysators oder Filters das 1 bis 6-fache, insbesondere das 2,0 - 2,5-fache der Laufzeit bei der Zufuhr von Dialysierflüssigkeit stromab des Dialysators oder Filters beträgt. Auf der Grundlage dieser Annahme kann der charakteristische Grenzwert für die Unterscheidung zwischen Prä- und Postdilution festgelegt werden. Es hat sich ferner gezeigt, dass die Laufzeit der Druckwellen bei Prä- und Postdilution keine eindeutige Korrelation mit der Förderrate der Substituatpumpe aufweist. Daher kann der Grenzwert unabhängig von der Pumprate vorgegeben werden. Im übrigen sind die Auswirkungen der Prä- und Postdilution auf die Laufzeit der Druckwellen unabhängig davon, ob die Blutbehandlungsvorrichtung beispielsweise als Hämodiafiltrations- oder Hämofiltrationsvorrichtung betrieben wird.

Prinzipiell ist die einmalige Erkennung von Prä- und Postdilution kurz vor dem Beginn der Blutbehandlung ausreichend. Es ist aber auch möglich, die Blutbehandlung kontinuierlich auf Prä- und Postdilution zu überwachen.

Die Laufzeitmessung erfolgt zweckmäßigerweise zwischen einem Anfangs- und einem Endwert. Der Anfangswert wird von der Erzeugung einer Druckpulswelle durch die Substituatpumpe festgelegt. Hierzu wird zweckmäßigerweise die Stellung des Rotors der Substituatpumpe beispielsweise mit einem Hallsensor erfasst. Die Laufzeitmessung endet, wenn im extrakorporalen Blutkreislauf die von der Substituatpumpe erzeugte Druckpulswelle eintrifft. Vorzugsweise wird als Laufzeitende das Maximum des gemessenen Drucksignals angenommen, das sich eindeutig identifizieren lässt. Da die Substituatpumpe mit jeder Umdrehung Druckpulswellen erzeugt, kann die Blutbehandlung kontinuierlich auf Prä- oder Postdilution überwacht werden.

In der Praxis kann das Ereignis für die Erzeugung der Druckwelle durch die Substitutatpumpe in dem Signal des Hallsensors, das die Rotorstellung der Substituatpumpe angibt, nicht exakt erkannt werden. Vielmehr ist die Laufzeit, die mit dem Hallsensorsignal ermittelt wird, grundsätzlich von der Förderrate der Substituatpumpe abhängig. Deshalb wird die gemessene Laufzeit für unterschiedliche Förderraten vorzugsweise mit Korrekturfaktoren korrigiert, die aus dem Abstand des Magneten des Hallsensors und dem Schlauchroller auf dem Pumprotor der Substituatpumpe einerseits und der Förderrate der Substituatpumpe andererseits berechnet wird. Wenn in der Praxis die Laufzeitmessung bei einer definierten Förderrate der Substituatpumpe vorgenommen wird, ist der Einfluss der Förderrate auf die Laufzeit relativ einfach zu kompensieren.

Um eine hohe Genauigkeit der Detektion von Prä- und Postdilution zu gewährleisten, sollte das gemessene Drucksignal frei von Störungen sein. In der Praxis werden die auf die Substituatpumpe zurückzuführenden Druckwellen aber von weiteren Druckwellen überlagert, die auf die Umschaltung der in den bekannten Blutbehandlungsvorrichtungen vorgesehenen Bilanzkammern und auf die zusätzlichen Pumpen zurückzuführen sind, die neben der Substituatpumpe vorgesehen sind, zu denen die Blutpumpe, die Ultrafiltrationspumpe und die Konzentratpumpe(n) zählen. Zur Vermeidung von Messfehlern wird daher der Dialysator oder Filter vorzugsweise vom Flüssigkeitssystem abgetrennt, wenn die Laufzeitmessung erfolgt. Dadurch verbleiben nur die Druckwellen der Substituat und Blutpumpe im gemessenen Drucksignal, wenn der extrakorporale Blutkreislauf nicht angehalten wird. Da die Frequenz der von der Blutpumpe erzeugten Druckwelle infolge der sehr viel höheren Drehzahl der Blutpumpe verglichen mit der Substituatpumpe sehr viel größer als die Frequenz der von der Substituatpumpe produzierten Druckwelle ist, kann die Druckwelle der Blutpumpe mit einem Tiefpassfilter eliminiert werden. Dadurch verschwinden auch andere Hochfrequenzstörungen. In der Praxis kann die Laufzeitmessung auch bei definierten Förderraten der Substituat- und Blutpumpe durchgeführt werden, damit die Druckwelle der Blutpumpe mit einem definierten Tiefpassfilter sicher eliminiert werden kann.

Ferner wird der Gleichspannungsanteil des gemessenen Drucksignals vorzugsweise eliminiert, um den relevanten Wechselspannungsanteil besser auswerten zu können.

Die Substituatpumpe ist vorzugsweise eine Okklusionspumpe, insbesondere Rollenpumpe, die eindeutig zu identifizierende Druckpulswellen erzeugt. Die Substituatpumpe kann aber auch jeder anderen Bauart sein. Allein entscheidend ist, dass von der Substituatpumpe ausgehende Druckpulswellen im extrakorporalen Blutkreislauf nachweisbar sind.

Die erfindungsgemäße Vorrichtung zur extrakorporalen Blutbehandlung verfügt über eine Einrichtung zur Detektion der Zufuhr von Substitutionsflüssigkeit stromauf oder stromab des Dialysators oder Filters. Diese Einrichtung kann aber grundsätzlich auch ein Zusatzgerät zu der extrakorporalen Blutbehandlungsvorrichtung sein.

Die Einrichtung zur Detektion der Prä- und Postdilution weist eine Überwachungseinheit zum Messen der Laufzeit der Druckwellen der Substituatpumpe und eine Auswerteinheit auf, die eine Unterscheidung zwischen der Zufuhr von Substitutionsflüssigkeit stromauf oder stromab des Dialysators oder Filters auf der Grundlage der Laufzeitmessung erlaubt.

Die meisten für die Überwachungs- und Auswerteinheit erforderlichen Komponenten sind im Allgemeinen in den bekannten Blutbehandlungsvorrichtungen bereits vorhanden. Beispielsweise kann auf den venösen Drucksensor zum Messen des Drucks im Blutkreislauf stromab des Dialysators oder Filters zurückgegriffen werden. Auch steht eine Mikroprozessorsteuerung zur Verfügung. Damit ist der apparative Aufwand für die Schaffung der Einrichtung zur Detektion der Prä- und Postdilution in einer Blutbehandlungsvorrichtung relativ gering.

Im Folgenden wird eine Ausführungsbeispiel des erfindungsgemäßen Verfahrens sowie der erfindungsgemäßen Blutbehandlungsvorrichtung unter Bezugnahme auf die Figuren näher erläutert.

Es zeigen:
- Figur 1: eine Vorrichtung zur extrakorporalen Blutbehandlung mit einer Einrichtung zur Detektion der Prä- und Postdilution in stark vereinfachter schematischer Darstellung,
- Figur 2: eine schematische Darstellung des Rotors der Substituatpumpe der Blutbehandlungsvorrichtung zusammen mit einem die Stellung des Pumpenrotors anzeigenden Magneten und
- Figur 3: den zeitlichen Verlauf des venösen Drucksignals und des Hallsignals.

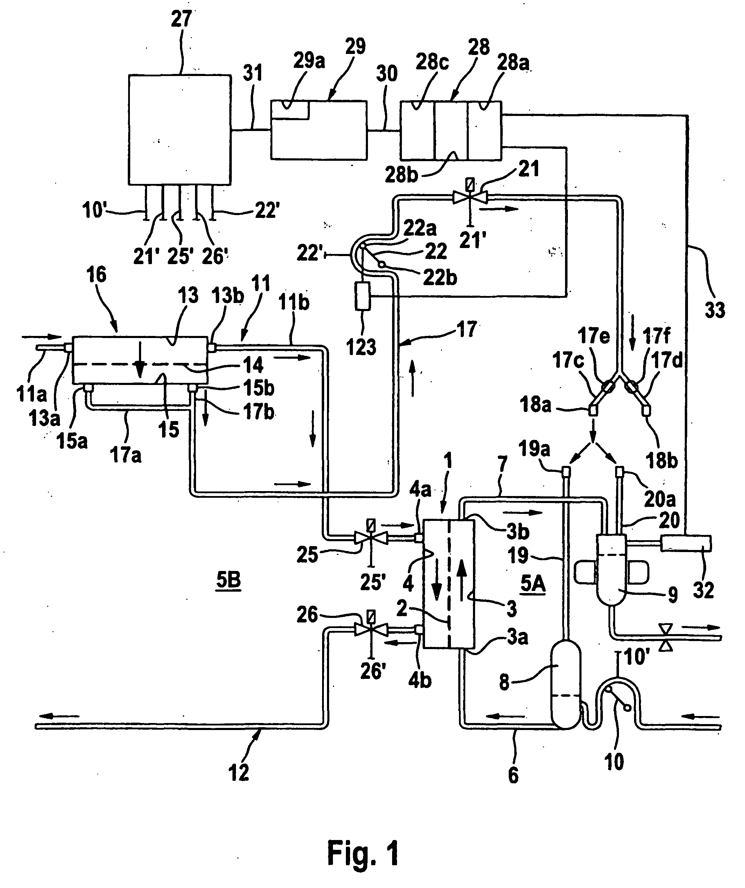

Figur 1 zeigt eine vereinfachte schematische Darstellung der wesentlichen Komponenten einer Hämo(dia)filtrationsvorrichtung zusammen mit einer Einrichtung zur Detektion der Zufuhr von Substitutionsflüssigkeit in den extrakorporalen Blutkreislauf stromauf oder stromab des Dialysators oder Filters. Wenn nachfolgend von einem Dialysator die Rede ist, wird darunter auch ein Filter verstanden.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Zur Eliminierung von Luftblasen sind in die arterielle Blutleitung 6 eine arterielle Tropfkammer 8 und in die venöse Blutleitung 7 eine venöse Tropfkammer 9 geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während die verbrauchte Dialysierflüssigkeit aus der Dialysierflüssigkeitskammer über die Dialysierflüssigkeitsabführleitung 12 zu einem nicht dargestellten Abfluss abgeführt wird. Die in den Hämo(dia)filtrationsvorrichtungen im Allgemeinen vorgesehene Bilanziereinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Die Dialysierflüssigkeitszuführleitung 11 umfasst einen ersten Abschnitt 11a, der zu dem Einlass 13a einer ersten Kammer 13 eines durch eine Membran 14 in die erste Kammer und eine zweite Kammer 15 unterteilten Sterilfilters 16 führt, und einen zweiten Abschnitt 11b, der von dem Auslass 13b der ersten Kammer 13 des Filters 16 abgeht und zu dem Einlass 4a der Dialysierflüssigkeitskammer 4 führt.

Während der Dialysebehandlung kann z. B. Dialysierflüssigkeit aus dem Flüssigkeitssystem 5B als Substitutionsflüssigkeit über eine Substitutionsflüssigkeitsleitung 17 dem extrakorporalen Blutkreislauf 5A zugeführt werden. Die Flüssigkeitsleitung 17 weist an beiden Enden jeweils zwei Leitungsabschnitte 17a, 17b, 17c, 17d auf. Der Leitungsabschnitt 17a ist mit dem ersten Auslass 15a und der Leitungsabschnitt 17b mit dem zweiten Auslass 15b der zweiten Kammer 15 des Sterilfilters 16 verbunden, während an den Leitungsabschnitten 17c und 17d jeweils ein Konnektor 18a, 18b angeschlossen ist. Mit den beiden Konnektoren 18a, 18b kann die Substitutionsflüssigkeitsleitung 17 an eine zu der arteriellen Tropfkammer 8 führende Anschlussleitung 19 bzw. eine zu der venösen Tropfkammer 9 führende Anschlussleitung 20 angeschlossen werden. Die Anschlussleitungen 19, 20 verfügen dafür über entsprechende Anschlussstücke 19a, 20a. Auf den Schlauchleitungsabschnitten 17c, 17d sitzen Schlauchklemmen 17e, 17f, mit denen wahlweise eine Flüssigkeitsverbindung nur zu der arteriellen oder venösen Tropfkammer 8, 9 hergestellt werden kann. Es ist aber auch möglich, das die Substitutionsflüssigkeitsleitung 17 mit beiden Anschlussleitungen 19, 20 verbunden und beide Schlauchklemmen 17e, 17f offen sind.

Zum Abklemmen der Substitutionsflüssigkeitsleitung 17 ist stromab des Sterilfilters 16 ein Absperrorgan 21, beispielsweise eine Schlauchklemme vorgesehen, die vorzugsweise elektromagnetisch betätigt wird. Die Substitutionsflüssigkeit wird mittels einer Okklusionspumpe, insbesondere Rollenpumpe 22 gefördert, in die die Substitutionsflüssigkeitsleitung 17 eingelegt ist. Derartige Rollenpumpen gehören zum Stand der Technik. Sie verfügen über mehrere Rollen 22a, 22b, mit denen der Querschnitt der Schlauchleitung zur Förderung der Flüssigkeit verringert werden kann. Dadurch entstehen Druckwellen, die sich in beiden Richtungen über die Substitutionsflüssigkeitsleitung fortpflanzen können. An der Substituatpumpe 22 ist ein Magnet vorgesehen, der in Kombination mit dem in der Maschine befindlichen Hall- Sensor 123 die Stellung des Pumpenrotors detektiert.

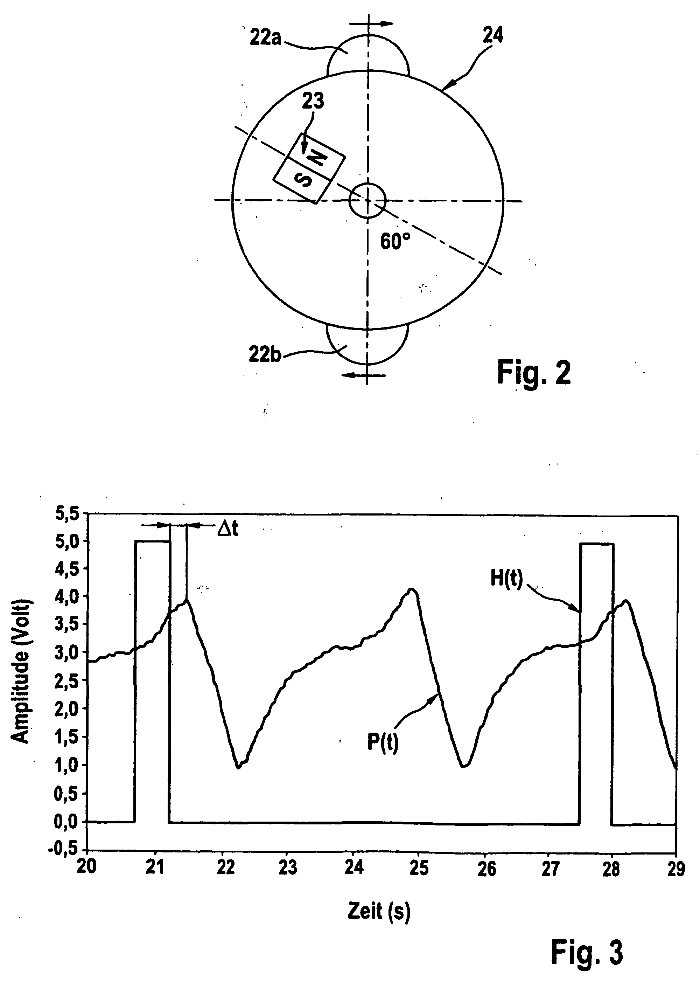

Figur 2 zeigt den Pumpenrotor 24 der Substituatpumpe zusammen mit dem Magneten, der mit jeder Umdrehung des Pumpenrotors ein rechteckförmiges Signal im Hall-Sensor 123 erzeugt.

Zur Abkopplung des Dialysators 1 von dem Flüssigkeitssystem 5B sind in der Dialysierflüssigkeitzuführleitung 11 stromauf und in der Dialysierflüssigkeitsabführleitung 12 stromab des Dialysators 1 jeweils ein elektromagnetisch betätigbares Absperrorgan 25, 26 vorgesehen.

Die Blutpumpe 10, die Substituatpumpe 22 sowie die Absperrorgane 21, 25 und 26 sind über Steuerleitungen 10', 22', 21', 25', und 26' mit einer zentralen Steuer- und Regeleinheit 27 verbunden, von der die einzelnen Komponenten unter Berücksichtigung der vorgegebenen Behandlungsparameter angesteuert werden.

Zum Betrieb der Hämo(dia)filtrationsvorrichtung als Hämodialysevorrichtung wird das Absperrorgan 21 geschlossen, wobei Dialysierflüssigkeit durch die Dialysierflüssigkeitskammer 4 des Dialysators strömt. Zum Betrieb der Hämo(dia)filtrationsvorrichtung als Hämodiafiltrationsvorrichtung wird das Absperrorgan 21 geöffnet, so dass aus dem Sterilfilter 16 sterile Dialysierflüssigkeit als Substituionsflüssigkeit wahlweise in die arterielle Tropfkammer 8 (Prädilution) oder venöse Tropfkammer 9 (Postdilution) strömt. Es ist aber auch ein Betrieb der Hämo(dia)filtrationsvorrichtung nur als Hämofiltrationsvorrichtung möglich, wenn der Zufluss von Dialysierflüssigkeit in die Dialysierflüssigkeitskammer 4 des Dialysators unterbrochen wird. Zur Unterbrechung der Flüssigkeitszufuhr wird das Absperrorgan 25 stromauf des Dialysators geschlossen.

Die Einrichtung zur Detektion der Prä- und Postdilution weist eine Überwachungseinheit 28 zum Messen der Laufzeit Δt der Druckwellen der Substituatpumpe 22 von der Substituatpumpe bis in den extrakorporalen Blutkreislauf 5A und eine Auswerteinheit 29 auf, die eine Prä- und Postdilution auf der Grundlage der Laufzeitmessung erkennt. Die Überwachungseinheit 28 und die Auswerteinheit 29 tauschen über eine Datenleitung 30 die entsprechenden Daten aus. Darüber hinaus stehen die Auswert- und Überwachungseinheit 28, 29 über eine Datenleitung 31 mit der zentralen Steuer- und Regeleinheit 27 in Verbindung.

Zum Messen des Drucks in der Blutabführleitung 7 stromab des Dialysators 1 ist an der Tropfkammer 9 ein venöser Drucksensor 32 vorgesehen, der ein von dem venösen Druck abhängiges elektrisches Signal erzeugt, das einen Gleich- und Wechselspannungsanteil hat. Figur 3 zeigt das venöse Drucksignal P(t) des Drucksensors 32 zusammen mit dem Signal H(t) des Hallsensors 123.

Der Drucksensor 32 ist über eine Signalleitung 33 mit der Überwachungseinheit 28 verbunden. Die Überwachungseinheit 28 weist Mittel 28a zum Eliminieren des Gleichspannungsanteils in dem Drucksignal, einen Verstärker 28b zum Verstärken des Drucksignals und einen Tiefpassfilter 28c zum Filtern des gemessenen Drucksignals auf. Zunächst wird der Gleichspannungsanteil von dem Wechselspannungsanteil getrennt. Daraufhin wird das Drucksignal auf einen bestimmten Spannungsbereich verstärkt, der beispielsweise zwischen 0 und 5V liegen kann. Schließlich wird der Wechselspannungsanteil des Drucksignals gefiltert, um Druckwellen der Blutpumpe und andere Hochfrequenzstörungen zu eliminieren.

Nachfolgend wird die Laufzeitmessung mit der Überwachungseinheit 28 erläutert. Zur Laufzeitmessung empfängt die Überwachungseinheit das Drucksignal P(t) des Drucksensors 32 und das Signal H(t) des Hallsensors 123. Die fallende Flanke des rechteckförmigen Signals setzt die Laufzeitmessung in Gang. Die Laufzeitmessung endet, wenn das Maximum der Druckpulswelle detektiert wird, die unmittelbar der fallenden Flanke des Rechtecksignals folgt. In Figur 3 ist die Laufzeit zwischen dem Signal H(t) und dem Drucksignal P(t) mit Δt bezeichnet.

Vor dem Start der eigentlichen Blutbehandlung schließt die Steuer- und Regeleinheit 27 die Absperrorgane 25, 26 stromauf bzw. stromab des Dialysators 1. Daraufhin setzt die Steuer- und Regeleinheit die Substituatpumpe 22 mit einer kleinen definierten Förderrate von beispielsweise 100ml/min für eine kurze Zeitdauer von beispielsweise 10 sek in Gang. Die Blutpumpe hingegen bleibt ausser Betrieb. Die Überwachungseinheit 28 misst die Laufeinheit Δt für jede Umdrehung des Rotors 24 der Substituatpumpe 22, wobei mit der Laufzeitmessung aber erst dann begonnen werden sollte, wenn sich eine Flüssigkeit, beispielsweise eine Spülflüssigkeit oder dgl. oder Blut im extrakorporalen Kreislauf befindet. Aus diesen Messwerten wird vorzugsweise ein Mittelwert gebildet. Dieser Mittelwert wird in der Auswerteinheit 29 mit einem vorgegebenen Grenzwert verglichen, der anhand von Probemessungen bei Prä- und Postdilution vorab ermittelt wird. Für den Fall, dass die Laufzeit den Grenzwert überschreitet, stellt die Auswerteinheit fest, dass die Substitutionsflüssigkeit stromauf des Dialysators 1 dem extrakorporalen Blutkreislauf zugeführt wird. Unterschreitet hingegen die Laufzeit den Grenzwert, stellt die Auswerteinheit fest, dass die Zufuhr der Substitutionsflüssigkeit stromab des Dialysators erfolgt. Auf einer Anzeige 29a zeigt die Auswerteinheit dann an, ob eine Prä- oder Postdilution vorliegt.

Die Überwachung auf Prä- oder Postdilution kann auch während der Blutbehandlung erfolgen. Hierzu wird zur Erhöhung der Genauigkeit aber der Dialysator von dem Dialysierflüssigkeitssystem abgekoppelt, ohne jedoch den extrakorporalen Kreislauf anzuhalten.

Nachfolgend wird die Auswertung der Laufzeit als Indiz für eine Prä- oder Postdilution anhand eines Beispiels erläutert.

Unter Verwendung eines Dialysegeräts der Fresenius AG mit der Typenbezeichnung 4008H wurden die oben genannten Bedingungen für die Laufzeitmessung simuliert.

Es wurde Rinderblut (Hämatokrit von 37%) sowohl für den extrakorporalen Blutkreislauf als auch für die Substitutionsflüssigkeit verwendet, um den Hämatokritwert des Blutes während der Messung zu stabilisieren. Das Signal des Hallsensors der Substituatpumpe und das venöse Drucksignal wurden mit Hilfe eines Datenerfassungssystems mit einer Auflösung von 16 Bit und einer Abtastfrequenz von 100 Hz aufgenommen, wobei die Rate der Substituatpumpe von 100 ml/min bis zu 350 ml/min im Abstand von 50 ml/min variiert wurde. Die Laufzeiten beim Verfahren mit Prä- bzw. Postdilution wurden offline berechnet und in der folgenden Tabelle zusammengestellt.

| Substituatpumpenrate (ml/min) | Laufzeit-Post (ms) | Laufzeit-Prä (ms) | Laufzeit-Prä / Laufzeit-Post |
|---|---|---|---|
| 100 | 90 | 217 | 2,41 |
| 150 | 93 | 205 | 2,20 |
| 200 | 100 | 230 | 2,30 |
| 250 | 103 | 210 | 2,04 |
| 300 | 100 | 210 | 2,10 |
| 350 | 92 | 217 | 2,36 |
| Mittelwert | 96,33 | 214,83 | 2,235 |
| Standardabweichung | 5,31 | 8,75 | 0,147 |

Aus der Tabelle ist eindeutig zu sehen:
- Die Laufzeit bei Prä- und Postdilution beträgt ca. 96 ms bzw. ca. 215 ms. Die Laufzeit bei Prädilution ist etwas mehr als zweimal größer als die Laufzeit bei Postdilution.
- Wird ein Schwellwert von 150 ms für die Erkennung festgelegt, ist von einer Postdilution auszugehen, wenn die gemessene Laufzeit kleiner als 150 ist. Ansonsten liegt eine Prädilution vor.

Wie oben erwähnt, wurde das Rinderblut sowohl für den extrakorporalen Blutkreislauf als auch für die Substitutionsflüssigkeit verwendet. Demgegenüber befindet sich das Blut bei einer wirklichen Dialysebehandlung nur im extrakorporalen Schlauchsystem, die Substitutionsflüssigkeitsleitung ist jedoch mit Substitutionsflüssigkeit gefüllt. Da die Viskosität von Blut etwa vierfach größer als die von Wasser ist, kann davon ausgegangen werden, dass die Laufzeit bei Postdilution noch kleiner als bei Prädilution ist, wodurch die Unterscheidung zwischen Prä- und Postdilution noch erleichtert wird.

Die unterschiedlichen Laufzeiten der Druckpulswellen bei Prä- und Postdilution sind darauf zurückzuführen, dass die Übertragungsstrecke der Druckpulswelle bei der Prädilution größer als bei der Postdilution ist. Das Verhältnis der Laufzeiten ist abhängig von der Länge der Schlauchleitungen für die Blutzu- und Abfuhr. In der Praxis ist die Laufzeit bei Prädilution mehr als dreimal so lang wie die Laufzeit bei Postdilution.

Die Substituatpumpe wird vorzugsweise mit einer definierten Förderrate von 100 ml/min und die Blutpumpe mit einer definierten Förderrate von 400 ml/min kurzzeitig für beispielsweise 10 sek betrieben. Da der Dialysator abgekoppelt ist, verbleiben nur die Druckwelle der Substituatpumpe und der Blutpumpe im gemessenen Drucksignal. Die Druckwelle der Blutpumpe wird vom Drucksignal mit dem Tiefpassfilter eliminiert, da der Hauptfrequenzanteil der Blutpumpe relativ weit vom Hauptfrequenzanteil der Substituatpumpe entfernt ist. In der Praxis ist der Hauptfrequenzanteil der Blutpumpe vier mal so groß wie der Frequenzanteil der Substituatpumpe.

Während der Dialysebehandlung kann die Laufzeitmessung in periodischen Abständen oder nur zu einem bestimmten Ereignis, beispielsweise beim Druckhaltetest der Hydraulik oder dem Konnektionstest im extrakorporalen Blutkreislauf durchgeführt werden.

Prinzipiell ist die Laufzeitmessung sowohl vor als auch während der Dialysebehandlung auch ohne die Abkopplung des Dialysators vom Dialysierflüssigkeitssystem möglich. Dies verlangt jedoch einen höheren Aufwand für die Kompensation von Störgrößen im Drucksignal.

In der folgenden Tabelle sind weitere Messergebnisse für unterschiedliche Hämatokritwerte dargestellt. Es zeigt sich, dass der Einfluss des Hämatokrit auf die Laufzeit bei Post- bzw. Prädilution zu vernachlässigen ist. Die unterschiedlichen Laufzeiten liegen für alle Hämatokritwerte in verschiedenen Wertebereichen, denen eindeutig eine Post- bzw. Prädilution zugeordnet werden kann.

| HKT | Subpumpen-rate (ml/min) | Laufzeit-Post (ms) | Laufzeit-Pre (ms) | Laufzeit-Pre/ -Post |
|---|---|---|---|---|
| 38 | 100 | 90 | 217 | 2,41 |
| | 150 | 93 | 205 | 2,20 |
| | 200 | 100 | 230 | 2,30 |
| | 250 | 103 | 210 | 2,04 |
| | 300 | 100 | 210 | 2,10 |
| | 350 | 92 | 217 | 2,36 |
| | Mittelwert (SD) | 96,33 (5,13) | 214,83 (8,75) | 2,24 |
| 34 | 100 | 110 | 228 | 2,07 |
| | 150 | 100 | 210 | 2,10 |
| | 200 | 100 | 205 | 2,05 |
| | 250 | 105 | 220 | 2,10 |
| | 300 | 105 | 220 | 2,10 |
| | 350 | 105 | 220 | 2,10 |
| | Mittelwert (SD) | 104,17 (3,76) | 217,17 (8,12) | 2,09 |
| 31 | 100 | 110 | 230 | 2,09 |
| | 150 | 120 | 230 | 1,92 |
| | 200 | 100 | 220 | 2,20 |
| | 250 | 100 | 210 | 2,10 |
| | 300 | 100 | 210 | 2,10 |
| | 350 | 100 | 215 | 2,15 |
| | Mittelwert (SD) | 105,00 (8,37) | 219,17 (9,03) | 2,09 |
| | Mittelwert | 102,01 | 217,06 | 2,14 |

## Patentansprüche

1. Verfahren zur Überwachung der Zufuhr von Substitutionsflüssigkeit in einem extrakorporalen Blutkreislauf, (5A) der eine erste Kammer (3) eines durch eine Membran (2) in die erste Kammer und eine zweite Kammer (4) unterteilten Dialysators (1) oder Filters einschließt, und einem Flüssigkeitssystem (5B) das die zweite Kammer des Dialysators oder Filters einschließt, wobei Substitutionsflüssigkeit stromauf oder stromab des Dialysators oder Filters mittels einer Druckwellen erzeugenden Substituatpumpe (22) dem extrakorporalen Blutkreislauf zugeführt wird, **dadurch gekennzeichnet, dass** für die Detektion der Zufuhr von Substitutionsflüssigkeit stromauf oder stromab des Dialysators oder Filters die Laufzeit Δt der Druckwellen der Substituatpumpe von der Substituatpumpe bis in den extrakorporalen Blutkreislauf gemessen wird, wobei eine Zufuhr von Substitutionsflüssigkeit stromauf oder stromab des Dialysators oder Filters auf der Grundlage der Laufzeitmessung erkannt wird.

2. Verfahren nach Anspruch 1 **dadurch gekennzeichnet, dass** zur Bestimmung der Laufzeit Δt der Druckwellen der Substituatpumpe (22) der Druck im extrakorporalen Kreislauf (5A) stromah des Dialysators (1) oder Filters gemessen wird.

3. Verfahren nach Anspruch 2, **dadurch gekennzeichnet, dass** die Laufzeit Δt der Druckwellen der Substituatpumpe (22) mit einem vorgegebenen Grenzwert verglichen wird, wobei bei Überschreiten des Grenzwertes auf eine Zufuhr von Substitutionsflüssigkeit stromauf des Dialysators (1) oder Filters und bei Unterschreiten des Grenzwertes auf eine Zufuhr stromab des Dialysator (1) oder Filters geschlossen wird.

4. Verfahren nach einem der Ansprüche 1 bis 3 **dadurch gekennzeichnet, dass** als Laufzeit Δt der Druckwellen der Substituatpumpe (22) bei einer Zufuhr von Substitutionsflüssigkeit stromauf des Dialysators (1) oder Filters das 1 bis 6-fache, vorzugsweise das 2,0 bis 2,5-fache der Laufzeit bei der Zufuhr von Substitutionsflüssigkeit stromab des Dialysators oder Filters angenommen wird.

5. Verfahren nach einem der Ansprüche 1 bis 4, **dadurch gekennzeichnet, dass** die Stellung des Rotors der Substituatpumpe (22) erfasst wird, wobei die Laufzeitmessung bei einer vorgegebenen Stellung des Pumpenrotors beginnt und bei der Detektion des resultierenden Druckpulswelle im extrakorporalen Blutkreislauf (5A) endet.

6. Verfahren nach Anspruch 5, **dadurch gekennzeichnet, dass** als Ende der Laufzeit ein charakteristischer Punkt eines Pulswellenereignisses, vorzugsweise das Maximum der Kurve eines Pulswellenereignisses angenommen wird.

7. Verfahren nach einem der Ansprüche 2 bis 6, **dadurch gekennzeichnet, dass** das gemessene Drucksignal mit einem Tiefpassfilter gefiltert wird.

8. Verfahren nach einem der Ansprüche 2 bis 7, **dadurch gekennzeichnet, dass** der Gleichspannungsanteil aus dem gemessenen Drucksignal eliminiert wird.

9. Verfahren nach einem der Ansprüche 1 bis 8, **dadurch gekennzeichnet, dass** der Dialysator (1) oder Filter vom Flüssigkeitssystem abgetrennt wird, wenn die Laufzeitmessung erfolgt.

10. Verfahren nach einem der Ansprüche 1 bis 9, **dadurch gekennzeichnet, dass** die Substituatpumpe (22) eine Okklusionspumpe, insbesondere Rollenpumpe ist.

11. Vorrichtung zur extrakorporalen Blutbehandlung mit einem extrakorporalen Blutkreislauf (5A), der eine erste Kammer (3) eines durch eine semipermeable Membran (2) in die erste Kammer und eine zweite Kammer (4) unterteilten Dialysators (1) oder Filters einschließt und einem Flüssigkeitssystem (5B), das die zweite Kammer des Dialysators oder Filters einschließt, wobei eine Substitutionsflüssigkeitsleitung (17) zu dem Blutkreislauf stromauf oder stromab des Dialysators oder Filters führt, in welcher Substitutionsflüssigkeitsleitung (17) eine Druckwellen erzeugende Substituatpumpe (22) angeordnet ist,
**gekennzeichnet durch**,
eine Einrichtung zur Detektion der Zufuhr von Substitutionsflüssigkeit stromauf oder stromab des Dialysators (1) oder Filters, die eine Überwachungseinheit (28) zum Messen der Laufzeit Δt der Druckwellen der Substituatpumpe (22) von der Substituatpumpe bis in den extrakorporalen Blutkreislauf (5A) und eine Auswerteinheit (29) aufweist, die derart ausgebildet ist, dass eine Zufuhr von Substitutionsflüssigkeit stromauf oder stromab des Dialysators oder Filters auf der Grundlage der Laufzeitmessung erkennbar ist.

12. Vorrichtung nach Anspruch 11, **dadurch gekennzeichnet, dass** Mittel (32) zum Messen des Drucks im extrakorporalen Blutkreislauf (5A) stromab des Dialysators (1) oder Filters vorgesehen sind.

13. Vorrichtung nach Anspruch 12, **dadurch gekennzeichnet, dass** die Auswerteinheit (29) Mittel zum Vergleichen der Laufzeit Δt der Druckwellen der Substituatpumpe (22) mit einem vorgegebenen Grenzwert aufweist, wobei die Auswerteinheit derart ausgebildet ist, dass bei Überschreiten des Grenzwertes auf eine Zufuhr von Substitutionsflüssigkeit stromauf des Dialysators (1) oder Filters und bei Unterschreiten des Grenzwertes auf eine Zufuhr stromab des Dialysators oder Filters geschlossen wird.

14. Vorrichtung nach einem der Ansprüche 11 bis 13, **dadurch gekennzeichnet, dass** als Laufzeit Δt der Druckwellen der Substituatpumpe (22) bei einer Zufuhr von Substitutionsflüssigkeit stromauf des Dialysators (1) oder Filters das 1 bis 6-fache, vorzugsweise das 2,0 bis 2,5-fache der Laufzeit Δt bei der Zufuhr von Substitutionsflüssigkeit stromab des Dialysators oder Filters angenommen wird.

15. Vorrichtung nach einem der Ansprüche 11 bis 14, **dadurch gekennzeichnet, dass** Mittel (23) zum Erfassen der Stellung des Rotors (24) der Substituatpumpe (22) vorgesehen sind, wobei die Überwachungseinheit (28) derart ausgebildet ist, dass die Laufzeitmessung bei einer vorgegebenen Stellung des Pumpenrotors beginnt und bei der Detektion der resultierenden Druckpulswelle im extrakorporalen Blutkreislauf (5A) endet.

16. Vorrichtung nach Anspruch 15, **dadurch gekennzeichnet, dass** die Überwachungseinheit (28) als Ende der Laufzeit Δt einen charakteristischen Punkt eines Pulswellenereignisses, vorzugsweise das Maximum der Kurve eines Pulswellenereignisses annimmt.

17. Vorrichtung nach einem der Ansprüche 12 bis 16, **dadurch gekennzeichnet, dass** die Überwachungseinheit (28) einen Tiefpassfilter (28c) zum Filtern des gemessenen Drucksignals aufweist.

18. Vorrichtung nach einem der Ansprüche 12 bis 17, **dadurch gekennzeichnet, dass** die Überwachungseinheit (28) Mittel (28a) zum Eliminieren des Gleichspannungsanteils des gemessenen Drucksignals aufweist.

19. Vorrichtung nach einem der Ansprüche 11 bis 18, **dadurch gekennzeichnet, dass** Mittel (25, 26) zum Abtrennen des Dialysators (1) oder Filters vom Flüssigkeitssystem (5B) vorgesehen sind.

20. Vorrichtung nach einem der Ansprüche 11 bis 19, **dadurch gekennzeichnet, dass** die Substituatpumpe (22) eine Okklusionspumpe, insbesondere Rollenpumpe ist.

## Claims

1. A method for monitoring the supply of substitution fluid in an extracorporeal blood circuit (5A), which includes a first chamber (3) of a dialyser (1) or filter divided by a membrane (2) into a first chamber and a second chamber (4), and a fluid system (5B), which includes the second chamber of the dialyser or filter, whereby the substitution fluid upstream or downstream of the dialyser or filter is fed to the extracorporeal blood circuit by means of a substituate pump (22) generating pressure waves, **characterised in that**, for the detection of the supply of substitution fluid upstream or downstream of the dialyser or filter, the propagation time At of the pressure waves of the substituate pump from the substituate pump up to the extracorporeal blood circuit is measured, whereby a supply of substitution fluid upstream or downstream of the dialyser or filter is detected on the basis of the propagation-time measurement.

2. The method according to claim 1, **characterised in that** the pressure in the extracorporeal circuit (5A) downstream of the dialyser (1) or filter is measured in order to determine propagation time Δt of the pressure waves of the substituate pump (22).

3. The method according to claim 2, **characterised in that** propagation time Δt of the pressure waves of the substituate pump (22) is compared with a preset limiting value, whereby a supply of substitution fluid upstream of the dialyser (1) or filter is deduced when the limiting value is exceeded and a supply downstream of the dialyser (1) or filter is deduced when the limiting value is fallen below.

4. The method according to any one of claims 1 to 3, **characterised in that** 1 to 6 times, preferably 2.0 to 2.5 times, the propagation time with the supply of substitution fluid downstream of the dialyser or filter is assumed as the propagation time Δt of the pressure waves of the substituate pump (22) with a supply of substitution fluid upstream of the dialyser (1) or filter.

5. The method according to any one of claims 1 to 4, **characterised in that** the position of the rotor of the substituate pump (22) is detected, whereby the propagation-time measurement begins with a preset position of the pump rotor and ends with the detection of the resulting pressure pulse wave in the extracorporeal blood circuit (5A).

6. The method according to claim 5, **characterised in that** a characteristic point of the pulse wave event, preferably the maximum of the curve of a pulse wave event, is adopted as the end of the propagation time.

7. The method according to any one of claims 2 to 6, **characterised in that** the measured pressure signal is filtered with a low-pass filter.

8. The method according to any one of claims 2 to 7, **characterised in that** the direct-voltage component is eliminated from the measured pressure signal.

9. The method according to any one of claims 1 to 8, **characterised in that** the dialyser (1) or filter is separated from the fluid system when the propagation-time measurement is taking place.

10. The method according to any one of claims 1 to 9, **characterised in that** the substituate pump (22) is an occlusion pump, in particular a roller pump.

11. An apparatus for extracorporeal blood treatment with
an extracorporeal blood circuit (5A), which includes a first chamber (3) of a dialyser (1) or filter divided by a semipermeable membrane (2) into the first chamber and a second chamber (4), and a fluid system (5B), which includes the second chamber of the dialyser or filter, whereby a substitution fluid line (17) leads to the blood circuit upstream or downstream of the dialyser or filter, in which substitution fluid line (17) there is arranged a substituate pump (22) generating pressure waves,
**characterised by**
a device for the detection of the supply of substitution fluid upstream and downstream of the dialyser (1) or filter, which device has a monitoring unit (28) for measuring propagation time At of the pressure waves of the substituate pump (22) from the substituate pump up to the extracorporeal blood circuit (5A) and an evaluation unit (29), which is designed in such a way that a supply of substitution fluid upstream or downstream of the dialyser or filter can be detected on the basis of the propagation-time measurement.

12. The apparatus according to claim 11, **characterised in that** means (32) are provided for measuring the pressure in the extracorporeal blood circuit (5A) downstream of the dialyser (1) or filter.

13. The apparatus according to claim 12, **characterised in that** the evaluation unit (29) has means for comparing propagation time Δt of the pressure waves of the substituate pump (22) with a preset limiting value, whereby the evaluation unit is designed in such a way that a supply of substitution fluid upstream of the dialyser (1) or filter is deduced when the limiting value is exceeded and a supply downstream of the dialyser or filter is deduced when the limiting value is fallen below.

14. The apparatus according to any one of claims 11 to 13, **characterised in that** 1 to 6 times, preferably 2.0 to 2.5 times, the propagation time Δt with the supply of substitution fluid downstream of the dialyser or filter is assumed as the propagation time Δt of the pressure waves of the substituate pump (22) with a supply of substitution fluid upstream of the dialyser (1) or filter.

15. The apparatus according to any one of claims 11 to 14, **characterised in that** means (23) are provided for detecting the position of the rotor (24) of the substituate pump (22), whereby the monitoring unit (28) is designed in such a way that the propagation-time measurement begins with a preset position of the pump rotor and ends when the resulting pressure pulse wave is detected in the extracorporeal blood circuit (5A).

16. The apparatus according to claim 15, **characterised in that** the monitoring unit (28) adopts a characteristic point of a pressure wave event, preferably the maximum of the curve of a pressure wave event, as the end of propagation time Δt.

17. The apparatus according to any one of claims 12 to 16, **characterised in that** the monitoring unit (28) has a low-pass filter (28c) for filtering the measured pressure signal.

18. The apparatus according to any one of claims 12 to 17, **characterised in that** the monitoring unit (28) has means (28a) for eliminating the direct voltage component of the measured pressure signal.

19. The apparatus according to any one of claims 11 to 18, **characterised in that** means (25, 26) are provided for separating the dialyser (1) or filter from the fluid system (5B).

20. The apparatus according to any one of claims 11 to 19, **characterised in that** the substituate pump (22) is an occlusion pump, in particular a roller pump.

## Revendications

1. Procédé pour surveiller l'arrivée de liquide de substitution dans une circulation sanguine extracorporelle (5A) qui comprend une première chambre (3) d'un dialyseur (1) ou d'un filtre subdivisé par une membrane (2) entre la première chambre et une seconde chambre (4), et dans un système de liquide (5B), qui comprend la seconde chambre du dialyseur ou du filtre, du liquide de substitution étant amené à la circulation sanguine extracorporelle en amont ou en aval du dialyseur ou du filtre au moyen d'une pompe à produit substitué (22) générant des ondes de pression, **caractérisé en ce que**, pour la détection de l'arrivée de liquide de substitution, le temps de propagation Δt des ondes de pression de la pompe à produit substitué depuis la pompe à produit substitué jusque dans la circulation sanguine extracorporelle est mesuré en amont ou en aval du dialyseur ou du filtre, une arrivée de liquide de substitution étant détectée en amont ou en aval du dialyseur ou du filtre sur la base de la mesure du temps de propagation.

2. Procédé selon la revendication 1, **caractérisé en ce que**, pour calculer le temps de propagation Δt des ondes de pression de la pompe à produit substitué (22), la pression dans la circulation sanguine (5A) extracorporelle est mesurée en aval du dialyseur (1) ou du filtre.

3. Procédé selon la revendication 2, **caractérisé en ce que** le temps de propagation Δt des ondes de pression de la pompe à produit substitué (22) est comparé avec une valeur limite prédéfinie, un dépassement de la valeur limite laissant conclure à une arrivée de liquide de substitution en amont du dialyseur (1) ou du filtre et un sous-dépassement de cette valeur limite à une arrivée en aval du dialyseur (1) ou du filtre.

4. Procédé selon l'une quelconque des revendications 1 à 3, **caractérisé en ce que**, comme temps de propagation Δt des ondes de pression de la pompe à produit substitué (22), pour une arrivée de liquide de substitution en amont du dialyseur (1) ou du filtre, on admet la multiplication par 1 à 6, de préférence par 2,0 jusqu'à 2,5, du temps de propagation lors de l'arrivée de liquide de substitution en aval du dialyseur ou du filtre.

5. Procédé selon l'une quelconque des revendications là 4, **caractérisé en ce que** la position du rotor de la pompe à produit substitué (22) est enregistrée, la mesure du temps de propagation commençant pour une position prédéfinie du rotor de pompe et se terminant lors de la détection de l'onde d'impulsion de pression qui en résulte dans la circulation sanguine (5A) extracorporelle.

6. Procédé selon la revendication 5, **caractérisé en ce qu'**un point caractéristique d'un événement d'impulsion d'onde, de préférence le maximum de la courbe d'un événement d'impulsion d'onde est admis comme fin du temps de propagation.

7. Procédé selon l'une quelconque des revendications 2 à 6, **caractérisé en ce que** le signal de pression mesurée est filtré avec un filtre passe-bas.

8. Procédé selon l'une quelconque des revendications 2 à 7, **caractérisé en ce que** la partie de tension continue est éliminée à partir du signal de pression mesurée.

9. Procédé selon l'une quelconque des revendications 1 à 8, **caractérisé en ce que** le dialyseur (1) ou le filtre est séparé du système de liquide lorsque la mesure du temps de propagation intervient.

10. Procédé selon l'une quelconque des revendications 1 à 9, **caractérisé en ce que** la pompe à produit substitué (22) est une pompe à occlusion, en particulier une pompe à rouleaux.

11. Dispositif pour le traitement extracorporel du sang avec
une circulation sanguine (5A) extracorporelle, qui comprend une première chambre (3) d'un dialyseur (1) ou filtre subdivisé par une membrane (2) semi-perméable entre la première chambre et une seconde chambre (4) et un système de liquide (5B), qui comprend la seconde chambre du dialyseur ou du filtre, une conduite pour liquide de substitution (17) aboutissant à la circulation sanguine en amont ou aval du dialyseur ou du filtre, conduite (17) dans laquelle est disposée une pompe à produit substitué (22) générant des ondes de pression,
**caractérisé en ce que**,
un appareil pour la détection de l'arrivée de liquide de substitution en amont ou en aval du dialyseur (1) ou du filtre, qui présente une unité de contrôle (28) pour la mesure du temps de propagation Δt des ondes de pressions de la pompe à produit substitué (22) depuis la pompe à produit substitué jusque dans la circulation sanguine (5A) extracorporelle et une unité d'analyse (29) qui est conçue de telle sorte qu'une arrivée de liquide de substitution est détectable en amont ou en aval du dialyseur ou du filtre sur la base de la mesure du temps de propagation.

12. Dispositif selon la revendication 11, **caractérisé en ce que** des moyens (32) sont prévus pour la mesure de la pression dans la circulation sanguine (5A) extracorporelle en aval du dialyseur (1) ou du filtre.

13. Dispositif selon la revendication 12, **caractérisé en ce que** l'unité d'analyse (29) présente des moyens pour la comparaison du temps de propagation Δt des ondes de pression de la pompe à produit substitué (22) avec une valeur limite prédéfinie, l'unité d'analyse étant conçue de telle sorte qu'un dépassement de la valeur limite permet de conclure à une arrivée de liquide de substitution en amont du dialyseur (1) ou du filtre et un sous-dépassement de la valeur limite à une arrivée en aval du dialyseur ou du filtre.

14. Dispositif selon l'une quelconque des revendications 11 à 13, **caractérisé en ce que**, comme temps de propagation Δt des ondes de pression de la pompe à produit substitué (22), pour une arrivée de liquide de substitution en amont du dialyseur (1) ou du filtre, on admet la multiplication par 1 jusqu'à 6, de préférence par 2,0 jusqu'à 2,5, du temps de propagation Δt en cas d'arrivée du liquide de substitution en aval du dialyseur ou du filtre.

15. Dispositif selon l'une quelconque des revendications 11 à 14, **caractérisé en ce que** des moyens (23) sont prévus pour l'enregistrement de la position du rotor (24) de la pompe à produit substitué (22), l'unité de contrôle (28) étant réalisée de telle sorte que la mesure du temps de propagation commence pour une position prédéfinie du rotor de pompe et se termine lors de- la détection de l'onde d'impulsion de pression qui en résulte dans la circulation sanguine (5A) extracorporelle.

16. Dispositif selon la revendication 15, **caractérisé en ce que** l'unité de contrôle (28) admet comme fin du temps de propagation Δt un point caractéristique d'un événement d'onde d'impulsion et de préférence le maximum de la courbe d'un événement d'onde d'impulsion.

17. Dispositif selon l'une quelconque des revendications 12 à 16, **caractérisé en ce que** l'unité de contrôle (28) présente un filtre passe-bas (28c) pour le filtrage du signal de pression mesuré.

18. Dispositif selon l'une quelconque des revendications 12 à 17, **caractérisé en ce que** l'unité de contrôle (28) présente des moyens (28a) pour l'élimination de la fraction de tension continue du signal de pression mesuré.

19. Dispositif selon l'une quelconque des revendications 11 à 18, **caractérisé en ce que** des moyens (25, 26) sont prévus pour la séparation du dialyseur (1) ou du filtre du système de liquide (5B).

20. Dispositif selon l'une quelconque des revendications 11 à 19, **caractérisé en ce que** la pompe à produit substitué (22) est une pompe à occlusion, en particulier une pompe à rouleaux.
